# EUROPEAN PATENT APPLICATION

(11) **EP 4 393 562 A1**
(43) Date of publication of application: **03.07.2024**
(21) Application number: 22217072.2
(22) Date of filing: 29.12.2022
(51) Int. Cl.: B01D 3/14, B01D 3/32, C07C 7/04, C07C 11/107, C07C 11/04

(54) **METHOD FOR PURIFYING LINEAR ALPHA OLEFINS WITH STAGE-HEATED DISTILLATION COLUMN**

(71) Applicant: SABIC Global Technologies B.V., 4612 PX Bergen op Zoom (NL)
(72) Inventor: Huckman, Michael Edward, 562125 Bengaluru (IN); MERENOV, Andrei, 562125 Bengaluru (IN)
(74) Representative: Hoeger, Stellrecht & Partner Patentanwälte mbB

(57) **Abstract**

The disclosure provides a method of purifying a linear alpha olefin product that includes feeding a linear alpha olefin feed stream comprising the linear alpha olefin product and ethylene into a feed stage of a distillation column, the distillation column having a plurality of stacked stages positioned between an overhead outlet and a bottoms reboiler including a stripping section and a rectifying section; feeding an aliphatic paraffin hydrocarbon solvent to the rectifying section of the distillation column to absorb linear alpha olefin; adding heat to at least one of said plurality of stacked stages in the stripping section of the distillation column between the feed stage and the bottoms reboiler; withdrawing an overhead stream comprising ethylene from the overhead outlet; and withdrawing a bottoms stream from the distillation column comprising the linear alpha olefin product.

## Description

### TECHNOLOGICAL FIELD

The present disclosure relates to methods for purifying a linear alpha olefin product stream from an oligomerization reaction.

### BACKGROUND

Linear olefins are a class of hydrocarbons useful as raw materials in the petrochemical industry and among these the linear alpha olefins, unbranched olefins whose double bond is located at a terminus of the chain, form an important subclass. Linear alpha olefins can be converted to linear primary alcohols by hydroformylation. Hydroformylation can also be used to prepare aldehydes, which in turn can be oxidized to afford synthetic fatty acids, especially those with an odd carbon number, useful in the production of lubricants. Linear alpha olefins are also used in the production of detergents, such as linear alkylbenzenesulfonates, which are prepared by Fiedel-Crafts reaction of benzene with linear olefins followed by sulfonation. Another important use of linear alpha olefins relates to production of linear low-density polyethylene (LLDPE) through catalytic co-polymerization with ethylene.

Preparation of alpha olefins is based largely on oligomerization of ethylene, which has a corollary that the alpha-olefins produced have an even number of carbon atoms. Oligomerization processes for ethylene mainly utilize organoaluminum compounds or transition metals as catalysts. Oligomerization methods are typically carried out in the presence of a catalyst that includes a zirconium component, such as zirconium tetraisobutyrate, and an aluminum component as activator, such as ethyl aluminum sesquichloride. Typically, the effluent from the reactor used to produce the linear alpha olefins is directed to one or more distillation columns to separate the various fractions of linear alpha olefins.

It is desirable to purify the alpha olefin products of oligomerization reactions to a very high purity level, such as a purity level of greater than 99.5 mol%. Achieving such a high degree of purity can be challenging using conventional separation processes due to the presence of reactor effluent containing components having widely varying boiling points. Due to this large range of boiling points, in a conventional distillation column, the temperature will change dramatically in the stripping section of the column, leading to a large sensible heat duty that makes efficient distillation column design difficult. There remains a need in the art for improvements in separation processes for such products.

### BRIEF SUMMARY

Example implementations of the present disclosure are directed to processes and systems for purifying a linear alpha olefin product stream, particularly a linear alpha olefin product stream that is a mixture of components with widely differing boiling points (e.g., a stream containing C2 through C10+ components). In particular, the processes and systems of the present disclosure utilize a stage-heated distillation column that adds heat directly to certain stages of the column so that all sensible heat needed in the column is not required from the reboiler. In this manner, efficient distillation column design and its stable operation is possible without varying the column diameter dramatically in the stripping section to account for steep liquid/vapor flow gradient. Still further, in certain embodiments, an aliphatic paraffin hydrocarbon solvent is added to the rectifying section of the distillation column to absorb linear alpha olefin and prevent or reduce loss of the desired product in the overhead product stream of the column, which improves performance of the column and avoids the need for an expensive refrigeration system that would be required if a conventional overhead condenser with reflux was used. The selected solvent is typically the same solvent used in an upstream oligomerization process to produce the linear alpha olefin, and the solvent stream to the column is typically recycled from a downstream separation process that recovers the solvent for reuse in the reactor.

The present disclosure includes, without limitation, the following embodiments.

Embodiment 1: A method of purifying a linear alpha olefin product, comprising: feeding a linear alpha olefin feed stream comprising the linear alpha olefin product and ethylene into a feed stage of a distillation column, the distillation column having a plurality of stacked stages positioned between an overhead outlet and a bottoms reboiler including a stripping section of the distillation column between the feed stage and the bottoms reboiler and a rectifying section between the feed stage and the overhead effluent; feeding an aliphatic paraffin hydrocarbon solvent (e.g., n-heptane) to the rectifying section of the distillation column to absorb linear alpha olefin; adding heat to at least one of said plurality of stacked stages in the stripping section of the distillation column between the feed stage and the bottoms reboiler; withdrawing an overhead stream comprising ethylene from the overhead outlet; and withdrawing a bottoms stream from the distillation column comprising the linear alpha olefin product (e.g., 1-hexene).

Embodiment 2: The method of Embodiment 1, comprising adding heat to a plurality of stacked stages in the stripping section of the distillation column, such as adding heat to at least 2 and up to 6 stages.

Embodiment 3: The method of Embodiment 1 or 2, wherein said adding heat comprises adding heat through a heat exchanger positioned either within the distillation column or external to the distillation column, and optionally wherein the amount of heat added to the stripping section above the reboiler is about 50 to about 75% of the total heat duty added to the stripping section of the distillation column.

Embodiment 4: The method of any one of Embodiments 1-3, wherein adding heat comprises withdrawing a liquid side stream from at least one of said plurality of stacked stages in the stripping section of the distillation column; feeding the side stream into a side reboiler adapted to at least partially vaporize the side stream to produce a vapor-containing effluent; and returning the vapor-containing effluent from the side reboiler to a stage of the stripping section of the distillation column.

Embodiment 5: The method of any one of Embodiments 1-4, further comprising conducting the (i) withdrawing, (ii) feeding, and (iii) returning steps at a plurality of stages in the stripping section of the distillation column using a plurality of side reboilers.

Embodiment 6: The method of any one of Embodiments 1-5, wherein the stages of the distillation column have a first diameter in the rectifying section and a second diameter in the stripping section of the distillation column, wherein the second diameter is larger than the first diameter, such as wherein the second diameter is no more than five times the first diameter.

Embodiment 7: The method of any one of Embodiments 1-6, wherein the average stage-to-stage change in vapor flow rate in the bottom five stages of the stripping section of the distillation column is no more than about 12% and/or the average stage-to-stage change in liquid flow rate in the bottom five stages of the stripping section of the distillation column is no more than about 20%.

Embodiment 8: The method of any one of Embodiments 1-7, wherein the overhead stream comprises about 90% by weight ethylene or greater, such as about 95% by weight ethylene or greater, and/or wherein a mass ratio of aliphatic paraffin hydrocarbon solvent added to the distillation column to ethylene in the overhead stream is about 0.15 to about 0.5, and/or wherein the overhead stream passes through a compressor.

Embodiment 9: The method of any one of Embodiments 1-8, wherein the linear alpha olefin feed stream comprises the aliphatic paraffin hydrocarbon solvent, such as n-heptane, and 1-hexene.

Embodiment 10: The method of any one of Embodiments 1-9, wherein the linear alpha olefin feed stream comprises about 3% by weight or less of C5 or smaller hydrocarbons other than ethylene.

Embodiment 11: A system for preparing and purifying a linear alpha olefin product, comprising an ethylene oligomerization reactor, the reactor producing an effluent comprising the linear alpha olefin product and ethylene; a distillation column in fluid communication with the effluent of the ethylene oligomerization reactor at a feed stage, the distillation column having a plurality of stacked stages positioned between an overhead outlet and a bottoms reboiler including a stripping section of the distillation column between the feed stage and the bottoms reboiler and a rectifying section between the feed stage and the overhead outlet; an aliphatic paraffin hydrocarbon solvent source in fluid communication with the rectifying section of the distillation column; and at least one heating device positioned to add heat to at least one stage in the stripping section of the distillation column between the feed stage and the bottoms reboiler.

Embodiment 12: The system of Embodiment 11, wherein the heating device comprises one or more side reboilers, each reboiler operatively positioned to receive and at least partially vaporize a liquid side stream from a different stage of the stripping section of the distillation column and return a vapor-containing effluent to the stripping section of the distillation column.

Embodiment 13: The system of Embodiment 11 or Embodiment 12, wherein the number of side reboilers is between 2 and 6.

Embodiment 14: The system of any one of Embodiments 11-13, wherein the stages of the distillation column have a first diameter in the rectifying section and a second diameter in the stripping section of the distillation column, wherein the second diameter is larger than the first diameter, such as wherein the second diameter is no more than five times the first diameter.

Embodiment 15: The system of any one of Embodiments 11-14, wherein an overhead stream from the distillation column comprises about 90% by weight ethylene or greater, such as about 95% by weight ethylene or greater; and/or the effluent from the ethylene oligomerization reactor comprises the aliphatic paraffin hydrocarbon solvent and 1-hexene; and/or the effluent from the ethylene oligomerization reactor comprises about 3% by weight or less of C5 or smaller hydrocarbons other than ethylene.

These and other features, aspects, and advantages of the present disclosure will be apparent from a reading of the following detailed description together with the accompanying figures, which are briefly described below. The present disclosure includes any combination of two, three, four or more features or elements set forth in this disclosure, regardless of whether such features or elements are expressly combined or otherwise recited in a specific example implementation described herein. This disclosure is intended to be read holistically such that any separable features or elements of the disclosure, in any of its aspects and example implementations, should be viewed as combinable, unless the context of the disclosure clearly dictates otherwise.

It will therefore be appreciated that this Brief Summary is provided merely for purposes of summarizing some example implementations so as to provide a basic understanding of some aspects of the disclosure. Accordingly, it will be appreciated that the above described example implementations are merely examples and should not be construed to narrow the scope or spirit of the disclosure in any way. Other example implementations, aspects and advantages will become apparent from the following detailed description taken in conjunction with the accompanying figures which illustrate, by way of example, the principles of some described example implementations.

### BRIEF DESCRIPTION OF THE FIGURES

Having thus described aspects of the disclosure in the foregoing general terms, reference will now be made to the accompanying figures, which are not necessarily drawn to scale, and wherein:
FIG. 1 is a block diagram of an ethylene oligomerization system according to an example implementation of the present disclosure;
FIG. 2 is a schematic representation of an example embodiment of a stage-heated distillation column according to the present disclosure;
FIG. 3 graphically illustrates the results of a simulation of the temperature profile of the example embodiment of a stage-heated distillation column from the Experimental section;
FIG. 4 graphically illustrates the results of a simulation of the temperature profile of the conventional distillation column from the Experimental section;
FIG. 5 graphically illustrates the results of a simulation of the vapor and liquid flow profile of the example embodiment of a stage-heated distillation column from the Experimental section; and
FIG. 6 graphically illustrates the results of a simulation of the vapor and liquid flow profile of the conventional distillation column from the Experimental section.

### DETAILED DESCRIPTION

Some implementations of the present disclosure will now be described more fully hereinafter with reference to the accompanying figures, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art. Like reference numerals refer to like elements throughout.

Unless specified otherwise or clear from context, references to first, second or the like should not be construed to imply a particular order. A feature described as being above another feature (unless specified otherwise or clear from context) may instead be below, and vice versa; and similarly, features described as being to the left of another feature else may instead be to the right, and vice versa. Also, while reference may be made herein to quantitative measures, values, geometric relationships or the like, unless otherwise stated, any one or more if not all of these may be absolute or approximate to account for acceptable variations that may occur, such as those due to engineering tolerances or the like.

All ranges disclosed herein are inclusive of the endpoints, and the endpoints are independently combinable with each other (e.g., ranges of "up to 25 wt. %, or, more specifically, 5 wt. % to 20 wt. %", is inclusive of the endpoints and all intermediate values of the ranges of "5 wt. % to 25 wt. %," etc.). "Combination" is inclusive of blends, mixtures, alloys, reaction products, and the like.

As used herein, unless specified otherwise or clear from context, the "or" of a set of operands is the "inclusive or" and thereby true if and only if one or more of the operands is true, as opposed to the "exclusive or" which is false when all of the operands are true. Thus, for example, "[A] or [B]" is true if [A] is true, or if [B] is true, or if both [A] and [B] are true. Further, the articles "a" and "an" mean "one or more," unless specified otherwise or clear from context to be directed to a singular form.

### Ethylene Oligomerization Process and System

Linear alpha olefins (LAOs) are olefins with a chemical formula CₓH₂ₓ, distinguished from other mono-olefins with a similar molecular formula by linearity of the hydrocarbon chain and the position of the double bond at the primary or alpha position. Linear alpha olefins comprise a class of industrially important alpha-olefins, including 1-butene, 1-hexene, 1-octene, 1-decene, 1-dodecene, 1-tetradecene, 1-hexadecene, 1-octadecene, and higher blends of C₂₀-C₂₄, C₂₄-C₃₀, and C₂₀-C₃₀ olefins. Linear alpha olefins are useful intermediates for the manufacture of detergents, synthetic lubricants, copolymers, plasticizers, and many other important products.

Existing processes for the production of linear alpha olefins typically rely on the oligomerization of ethylene. For example, linear alpha olefins can be prepared by the catalytic oligomerization of ethylene in the presence of a Ziegler-Natta-type catalyst or non-Ziegler-Natta-type catalyst.

Oligomerization can occur at temperatures of 10 to 200° C, for example, 20 to 100° C, for example, 50 to 90° C, for example, 55 to 80° C, for example, 60 to 70° C. Operating pressures can be 1 to 5 MegaPascals (MPa), for example, 2 to 4 MPa. The process can be continuous and mean residence times can be 10 minutes to 20 hours, for example 30 minutes to 4 hours, for example, 1 to 2 hours. Residence times can be chosen so as to achieve the desired conversion at high selectivity.

The process can be conducted in solution using an inert solvent system, which can consist of one or more solvents, which are advantageously non-reactive with the catalyst composition. Examples of desirable organic solvents can include, but are not limited to, aromatic hydrocarbon solvents which can be unsubstituted or substituted with halogens, for example, toluene, benzene, xylene, monochlorobenzene, dichlorobenzene, chlorotoluene; aliphatic paraffin hydrocarbons, for example, pentane, hexane, heptane, octane, nonane, decane; alicyclic hydrocarbon compounds, for example, cyclohexane, decahydronaphthalene; and halogenated alkanes, for example, dichloroethane and dichlorobutane, and combinations thereof.

The process can be carried out in any reactor, such as a loop reactor, a plug-flow reactor, or a bubble column reactor. Oligomerization of ethylene is an exothermic reaction that can be cooled by a surplus flow of ethylene. The gases leaving at a top portion of the reactor can be cooled using a series of external coolers and condensers. The gas phase, after further cooling, can be recycled.

A bottom stream leaving the oligomerization reactor from a bottom portion can contain the active catalyst and unreacted ethylene. The reaction can be terminated to avoid undesirable side reactions by removing catalyst components from the organic phase through extraction with a caustic aqueous phase. Contact with the caustic aqueous phase can result in formation of nonreactive minerals corresponding to the catalyst components.

The organic phase, after passage through the catalyst removal system, can pass through a molecular sieve absorption bed and can then be fed to a distillation column to recover dissolved ethylene. Recovered ethylene can be recycled via an ethylene recycle loop while the product is fed to an intermediate vessel, after which the product can be fed to a separation section. In certain embodiments, the linear alpha olefins produced from the reactor can be directed into a separation train.

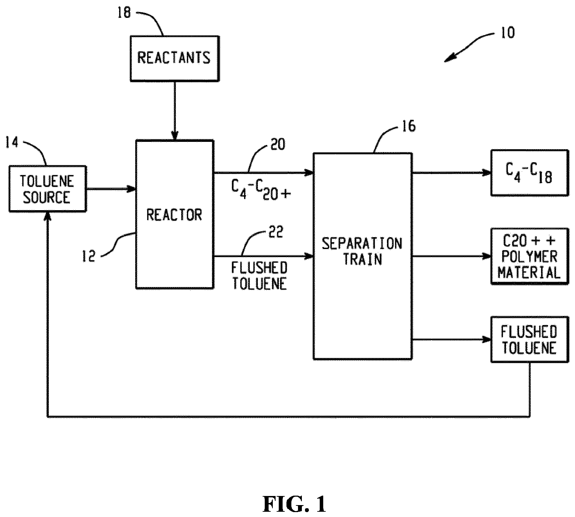

As illustrated in FIG. 1, the system 10 can include a reactor 12, a solvent source 14, and a separation train 16. In normal production mode, reactants 18, such as ethylene, solvent, and a catalyst can be fed into the reactor 12 to produce linear alpha olefins and various impurities such as branched olefins and polymeric material. After the reaction, a discharge stream 20 can be directed into the separation train 16, wherein the discharge stream can include unreacted reactants, the produced linear alpha olefins, such as C₄-C₂₀+ olefins, solvent, catalyst, and various impurities. The separation train 16 can be configured to separate the linear alpha olefins from the solvent, catalyst, various impurities, and any unreacted ethylene. The separation train 16 can separate each linear alpha olefin, for example, yielding a C₄ stream, C₆ stream, C₈ stream, and so on. The separation train 16 can also separate the linear alpha olefins into certain fractions, such as C₄-C₁₀ fraction, C₁₁-C₁₇ fraction, C₁₈-C₂₀ fraction, C₂₀₊ fractions, or any other desired fraction.

The linear alpha olefin product can be isolated using procedures including aqueous caustic catalyst quench followed by water washing and final product recovery by distillation. For example, the liquid product including the solvent with the dissolved ethylene can be fed to the separation train 16 as noted above. In a first column, the unconsumed ethylene can be separated from the linear alpha olefin product and the solvent. The ethylene can be recycled back to the reactor. The heavier fractions can be routed to the subsequent separation section where the heavier fractions can be divided into the different linear alpha olefin fractions (e.g., C8, C10, >C12). The solvent can be recovered and also recycled back to the reactor.

Polymer fouling within the reactor can occur during the oligomerization reaction process. Such fouling is typically detected by, for example, reduced effluent flow rates, reduced internal condenser performance, increased differential pressure at various locations within the reactor, and the like. Such fouling can be treated by flushing the reactor with solvent to remove polymeric material by-product. The flushed solvent comprising the polymeric material can be directed into a separation train comprising the linear alpha olefin reaction products. The polymeric material is soluble in at least one of the linear alpha olefins, such that the flushed solvent can exit the separation train essentially free of the polymeric material and can be recycled back to the solvent source for subsequent flushing of the reactor.

In certain embodiments, the selection of solvent system for the ethylene oligomerization reaction can increase selectivity for certain desirable linear alpha olefins. For example, it has been discovered that combination of a first paraffinic solvent with a second aromatic solvent can be advantageous for selective oligomerization. In certain embodiments adapted for selective production of 1-hexene, n-heptane is used as a first (paraffinic) solvent for boosting the selectivity of 1-hexene, optionally with xylene as a second (aromatic) solvent for catalyst dissolving and reactor cleaning.

Described oligomerization can lead to challenges separating unreacted ethylene from the reactor effluent due to a wide range of boiling points represented in the effluent. For example, where the oligomerization reaction is insufficiently selective for 1-hexene, the resulting effluent will include ethylene as well as C8+ components, leading to difficulty in stripping of ethylene from the reactor effluent in an initial distillation column of the separation train 16. The wide range of boiling points, coupled with a dearth of reactor effluent components with a chain length between ethylene and 1-hexene, results in the need for a large sensible heat duty to accomplish the desired ethylene stripping.

Typically, the reactor effluent is predominantly aliphatic paraffin hydrocarbon solvent, such as n-heptane (e.g., greater than 50 wt.% aliphatic paraffin hydrocarbon solvent), and also contains a linear alpha olefin, such as 1-hexene. In certain embodiments, the reactor effluent meets one or more of the following criteria: (1) more than about 7.5 wt.% (e.g., more than about 8 wt.% or more than about 9 wt.% or more than about 10 wt.%, such as about 7.5 wt.% to about 15 wt.%) of the effluent is ethylene; (2) more than about 60 wt.% (e.g., more than about 62 wt.% or more than about 65 wt.% or more than about 70 wt.%, such as about 60 wt.% to about 80 wt.%) of the effluent is aliphatic paraffin hydrocarbon solvent, such as n-heptane; (3) more than about 7.5 wt.% (e.g., more than about 8 wt.% or more than about 9 wt.% or more than about 10 wt.%, such as about 7.5 wt.% to about 15 wt.%) of the effluent is 1-hexene or another linear alpha olefin; and/or (4) less than about 3 wt.% (e.g., less than about 2 wt.% or less than about 1 wt.% or less than about 0.5 wt.%, such as about 0.1 wt.% to about 3 wt.%) of the effluent consists of C5 or smaller hydrocarbons other than ethylene.

### Stage-Heated Distillation Column

According to the present disclosure, the separation train 16 will include a first stage-heated distillation column adapted to recover unreacted ethylene from the reactor effluent. The type of distillation column may vary, with examples including columns with trays (bubble cap trays, valve trays, sieve trays etc.), or random or structured packing. The stage-heated distillation column is characterized by heat addition at one or more stages in the stripping section of the column to augment the heat provided by the reboiler. The heat addition is typically accomplished using a heat exchanger or other heating device, positioned either within the distillation column or external to the distillation column. The number of stages where heat is added can vary, but is typically 1 to 6 stages.

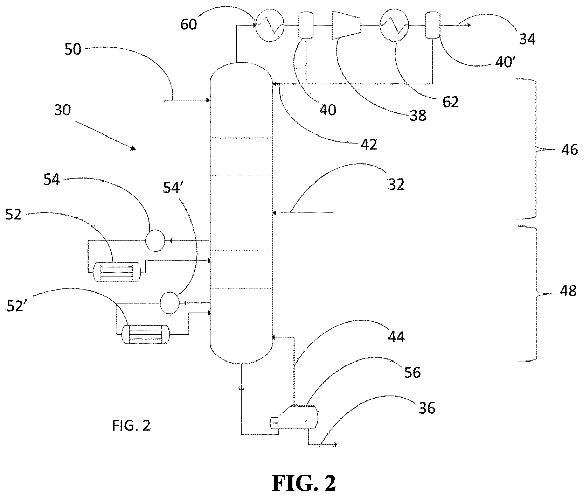

An example implementation of a stage-heated distillation column 30 is shown in FIG. 2. The distillation column 30 is in fluid communication with effluent from reactor 12 of FIG. 1, which is shown as feed stream 32 entering the column at a feed stage, the location of which may vary. The stage-heated distillation column 30 is adapted to separate the reactor effluent into an ethylene stream 34 suitable for recycle to the reactor 12 and a bottoms product stream 36 comprising the remainder of the reactor effluent including the desired linear alpha olefin and solvent. The bottoms product stream 36 can be further processed as desired to separate the remaining components of the reactor effluent. The bottoms product stream 36 is drawn from a reboiler 56, which also produces a boilup stream 44 returned to the column 30.

In some embodiments of the present disclosure, a conventional overhead condenser producing a reflux stream is too costly and complex to implement. A typical overhead effluent stream is predominately ethylene, which means an overhead condenser would require a refrigeration system in order to produce reflux to return to the column. To overcome this problem, in certain embodiments of the present disclosure, a solvent stream is fed to the rectifying section for adsorption of heavy components as explained more fully below. Accordingly, in certain embodiments, the system and method of the present disclosure can be characterized by an absence of an overhead condenser returning reflux to the column 30.

In certain embodiments, the overhead effluent from the distillation column 30 will be compressed in a compressor 38, which can be a multi-stage compressor having, for example 2 to 6 or 2 to 4 stages, prior to recycle back to the reactor. To improve the efficiency of the compression and remove condensate, the overhead effluent is optionally cooled in a heat exchanger 60 and passed through a vapor-liquid separator 40 upstream of the compressor 38. Optionally, the compressed overhead stream is also cooled following compression with heat exchanger 62 and also passed through a second vapor-liquid separator 40' to remove condensate prior to recycle. The combined condensate from the two vapor-liquid separators, 40 and 40', can be returned to the column 30 as a small reflux stream 42. Example vapor-liquid separators include flash drums, knock-out drums, knock-out pots, compressor suction drums, and the like.

In embodiments of the present disclosure, the stage-heated distillation column 30 comprises a rectifying section 46 above the feed stream 32 adapted to absorb 1-hexene or other linear alpha olefin from the reactor effluent and a stripping section 48 below the feed entry point where ethylene is stripped from the reactor effluent. In certain embodiments, at least a portion of the rectifying section 46 will also include an absorption section where heavier components including 1-hexene or other linear alpha olefin are removed from the stripped ethylene by contacting it with the aliphatic paraffin hydrocarbon solvent, typically the same solvent used in the reactor 12 of FIG. 1, which reduces or eliminates linear alpha olefin product from leaving in the overhead and being destroyed in the reactor. This absorption results from addition of an aliphatic paraffin hydrocarbon solvent (e.g., n-heptane) stream 50 to the column 30 proximal to the top of the column. The stage at which the aliphatic paraffin hydrocarbon solvent is added can vary, but is typically one of the top 5 stages of the column 30. The aliphatic paraffin hydrocarbon solvent stream 50 is typically obtained by recycle from a downstream portion of the separation train 16.

The amount of aliphatic paraffin hydrocarbon solvent added to the column 30 will vary, depending in part on the number of rectifying stages in the column. The amount of solvent needed will increase as the number of rectifying stages decreases. Typically, the column 30 will have 10 to 120 total stages, more typically about 20 to about 50 stages. The rectifying section of the column 30 will typically include about 5 to about 30 stages, such as about 5 to about 20 stages. The amount of solvent added to the column in the rectifying section can be characterized as a mass ratio of solvent added to ethylene in the overhead effluent from the column. An example range of solvent to ethylene mass ratio is about 0.15 to about 0.5, such as about 0.2 to about 0.4. In certain embodiments, the mass ratio is about 0.15 or higher, or about 0.2 or higher, or about 0.25 or higher, or about 0.3 or higher.

In certain embodiments of the present disclosure, the recycled ethylene stream 34 comprises about 90% by weight ethylene or greater, such as about 95% by weight ethylene or greater (e.g., about 97 to 99 wt.% ethylene), which can be compressed and returned to reactor 12. A small purge stream of the recycled ethylene stream (not shown) can be sent to a flare system in order to prevent accumulation of lighter components such as nitrogen in the process. The bottoms product stream 36 typically comprises very little ethylene, such as 1 to 3 wt.% ethylene.

The stage-heated distillation column 30 adds heat directly to certain stages of the stripping section 48 of the column so that all sensible heat needed in the column is not required from the reboiler 56. As noted above, this heat can be added through heat exchange that occurs either internally within the column 30 or external to the column. In this manner, efficient distillation column design is possible without multiple, radical changes in column diameter.

For purposes of illustration only, FIG. 2 shows external heat addition using heat exchangers 52, 52' and pumps 54, 54'. As shown, liquid can be withdrawn from a stage within the stripping section 48 of the distillation column 30 using a pump 54, 54' and passed through a heat exchanger 52, 52' which is adapted to introduce heat into the liquid withdrawn from the column, typically resulting in at least partial vaporization of the liquid. The vapor-containing effluent from the heat exchange is returned to the column 30. The location of the stage from which the liquid is withdrawn can vary. The vapor-containing effluent from the heat exchanger 52, 52' can be returned to the same stage from which liquid was withdrawn, or returned to a separate stage in the stripping section 48 of the column 30. Although FIG. 2 shows two heat exchangers 52, 52' withdrawing from two different stages of the column 30, as noted previously, the exact number of stages where heat is added can vary.

The amount of heat added to augment the reboiler heat duty can vary. The amount of heat added in the stripping section of the column 30 above the reboiler can be characterized as a percentage of the total heat duty added to the column (including all side reboilers and the main bottoms reboiler). In certain embodiments, the percentage of heat added above the bottoms reboiler (e.g., through side reboilers) is about 50 to about 75% of the total heat duty added, such as about 55 to about 70%. For example, if the total heat duty is about 6,500 to about 7,500 kW, the amount of heat added above the bottoms reboiler is typically about 3,250 to about 5,625 kW, such as about 3,575 to about 5,250 kW.

Due to the addition of heat in the stripping section to augment the reboiler, an efficient distillation column 30 can be designed, in certain embodiments, with only one tray diameter change across the length of the column. For example, the trays of the distillation column can have a first diameter in the rectifying section and a second diameter in the stripping section of the distillation column, wherein the second diameter is larger than the first diameter, such as no more than five times the first diameter or no more than four times the first diameter or no more than three times the first diameter. In other words, the ratio of the first diameter (rectifying section) and the second diameter (stripping section) is about 1:5 or less or about 1:4 or less or about 1:3 or less (e.g., a ratio of about 1:5 to about 1:2) . In certain embodiments, the entire rectifying section has the same first tray diameter and the entire stripping section has the same second tray diameter.

In certain embodiments, the distillation column 30 can be characterized by stage-to-stage stability in terms of temperature profile, as well as vapor and liquid flow profile, within the stripping section of the column. For example, in certain embodiments, the bottom five stages of the stripping section of the distillation column have a stage-to-stage change in temperature of about 2.5 °C or less or about 2.0 °C or less.

Additionally, in certain embodiments, the average stage-to-stage change in vapor flow rate in the bottom five stages of the stripping section of the distillation column is no more than about 12% (e.g., no more than 10% or no more than about 8%). This is calculated by determining the absolute percentage change (increase or decrease) in flow rate between each of the bottom five stages and averaging the total. In certain embodiments, the average stage-to-stage change in vapor flow rate in the bottom five stages of the stripping section of the distillation column is about 8,000 kg/hr or less. In some embodiments, the average stage-to-stage change in liquid flow rate in the bottom five stages of the stripping section of the distillation column is no more than about 20% (e.g., no more than 18% or no more than about 16%). This is calculated by determining the absolute percentage change (increase or decrease) in flow rate between each of the bottom five stages and averaging the total. In certain embodiments, the average stage-to-stage change in liquid flow rate in the bottom five stages of the stripping section of the distillation column is about 20,000 kg/hr or less.

### EXPERIMENTAL

Process simulation studies were performed using Aspen Plus. The simulation compared two distillation column designs: (1) a convention distillation column with 30 stages and with all heat added in the reboiler (stage 30); and (2) an inventive stage-heated distillation column with 30 stages and heat added at stages 19, 21, 24, and 27 in addition to the reboiler. In both cases, stage 18 was the feed stage and recycled n-heptane solvent was added at stage 3. The simulations used the same feed stream and the same target effluent streams for each column design. The feed stream characteristics are set forth in Table 1 below and the recycled n-heptane solvent characteristics are set forth in Table 2 below.

**TABLE 1**

| | |
|---|---|
| Flow (Kg/Hr) | 52,835 |
| Temp. (deg. C) | 70 |
| Pressure (Barg) | 30 |
| Mass Vapor Fraction | 0.02 |
| | |

| **Feed Components** | **mass fraction** |
|---|---|
| Methane | 0.0037 |
| Ethylene | 0.1016 |
| 1-Butene | 0.0042 |
| 1-Hexene | 0.0948 |
| Heptane | 0.7470 |

**TABLE 2**

| | |
|---|---|
| Flow (Kg/Hr) | 1,500 |
| Temp. (deg. C) | 46 |
| Pressure (Barg) | 31 |
| Mass Vapor Fraction | 0.00 |
| | |

| **Feed Component** | **mass fraction** |
|---|---|
| Heptane | 0.9998 |

Aspen Plus designed the most efficient column for each design case to achieve an overhead stream of at least 90% by weight ethylene. For the conventional column with reboiler, the Aspen Plus design included significant stage diameter changes across the column to account for the changing vapor and liquid flows, particularly in the stripping section. The simulated conventional column had three different diameters across the column, with a diameter of about 0.5 meters in the rectifying section (stages 1-17), and two distinct diameters in the stripping section. From stages 18 to 21, the required stage diameter was about 1.2 meters and from stages 22-29, the required diameter was about 3 meters. Thus, the conventional column design would need to be complex with significant diameter changes and at least three distinct sections.

In contrast, the simulation determined that the column design with additional heat added to four stages of the stripping section would be less complex, with a rectifying section having a diameter of about 0.5 meters (stages 1-17), and with a stripping section that could be optimized to a single diameter of approximately 1.8-2.2 meters. Unlike the conventional column design, the stage-heated column could be designed with only one diameter change across the length of the column.

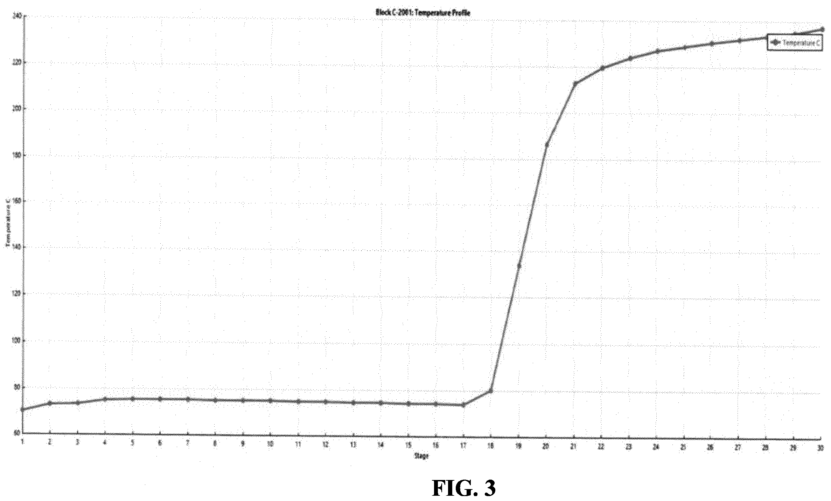

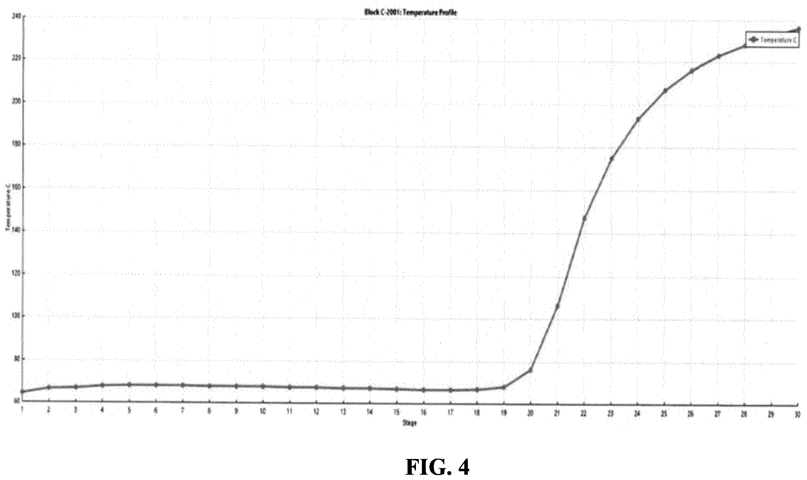

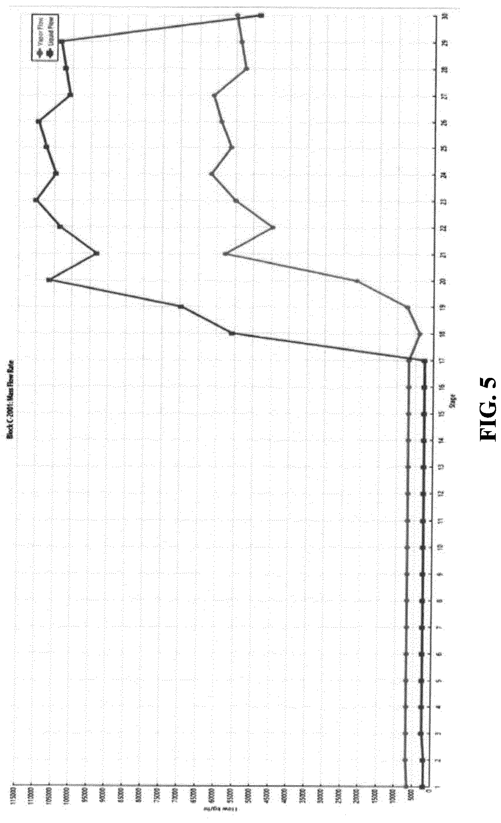

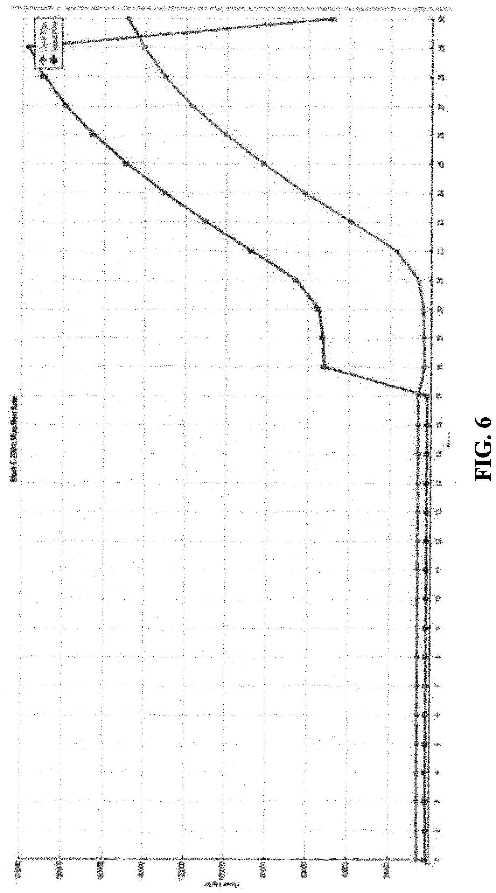

The simulation also calculated a temperature profile and a vapor/liquid flow profile for each column design. These profiles are set forth in FIGS. 3-6. As show in FIG. 3, for the stage-heated column, the temperature profile in the stripping section of the column (right-hand side of plot) is more stable from stage to stage as compared to the conventional column design (FIG. 4). Similarly, as show in FIG. 5, for the stage-heated column, the liquid and vapor flows in the stripping section of the column (right-hand side of plot) is more stable from stage to stage as compared to the conventional column design (FIG. 6). This increased stability of temperature and flow in the stripping section of the inventive column allows for an efficient column design and operation with only a single diameter change across the column.

In general, the invention may alternately comprise, consist of, or consist essentially of, any appropriate components herein disclosed. The invention may additionally, or alternatively, be formulated so as to be devoid, or substantially free, of any components, materials, ingredients, adjuvants or species used in the prior art compositions or that are otherwise not necessary to the achievement of the function and/or objectives of the present invention.

Many modifications and other implementations of the disclosure will come to mind to one skilled in the art to which this disclosure pertains having the benefit of the teachings presented in the foregoing descriptions and the associated figures. Therefore, it is to be understood that the disclosure is not to be limited to the specific implementations disclosed herein and that modifications and other implementations are intended to be included within the scope of the appended claims. Although specific terms are employed herein, they are used in a generic and descriptive sense only and not for purposes of limitation.

## Claims

1. A method of purifying a linear alpha olefin product, comprising:
(a) feeding a linear alpha olefin feed stream comprising the linear alpha olefin product and ethylene into a feed stage of a distillation column, the distillation column having a plurality of stacked stages positioned between an overhead outlet and a bottoms reboiler including a stripping section of the distillation column between the feed stage and the bottoms reboiler and a rectifying section between the feed stage and the overhead effluent;
(b) feeding an aliphatic paraffin hydrocarbon solvent to the rectifying section of the distillation column to absorb linear alpha olefin;
(c) adding heat to at least one of said plurality of stacked stages in the stripping section of the distillation column between the feed stage and the bottoms reboiler;
(d) withdrawing an overhead stream comprising ethylene from the overhead outlet; and
(e) withdrawing a bottoms stream from the distillation column comprising the linear alpha olefin product.

2. The method of claim 1, comprising adding heat to a plurality of stacked stages in the stripping section of the distillation column, such as adding heat to at least 2 and up to 6 stages.

3. The method of claim 1 or claim 2, wherein said adding heat comprises adding heat through a heat exchanger positioned either within the distillation column or external to the distillation column, and optionally wherein the amount of heat added to the stripping section above the reboiler is about 50 to about 75% of the total heat duty added to the stripping section of the distillation column.

4. The method of claim 1 or claim 2, wherein adding heat comprises:
i) withdrawing a liquid side stream from at least one of said plurality of stacked stages in the stripping section of the distillation column;
ii) feeding the side stream into a side reboiler adapted to at least partially vaporize the side stream to produce a vapor-containing effluent; and
iii) returning the vapor-containing effluent from the side reboiler to a stage of the stripping section of the distillation column.

5. The method of claim 4, further comprising conducting the (i) withdrawing, (ii) feeding, and (iii) returning steps at a plurality of stages in the stripping section of the distillation column using a plurality of side reboilers.

6. The method of claim 1 or claim 2, wherein the stages of the distillation column have a first diameter in the rectifying section and a second diameter in the stripping section of the distillation column, wherein the second diameter is larger than the first diameter, such as wherein the second diameter is no more than five times the first diameter.

7. The method of claim 1 or claim 2, wherein the average stage-to-stage change in vapor flow rate in the bottom five stages of the stripping section of the distillation column is no more than about 12% and/or the average stage-to-stage change in liquid flow rate in the bottom five stages of the stripping section of the distillation column is no more than about 20%.

8. The method of claim 1 or claim 2, wherein the overhead stream comprises about 90% by weight ethylene or greater, such as about 95% by weight ethylene or greater, and/or wherein a mass ratio of aliphatic paraffin hydrocarbon solvent added to the distillation column to ethylene in the overhead stream is about 0.15 to about 0.5, and/or wherein the overhead stream passes through a compressor.

9. The method of claim 1 or claim 2, wherein the linear alpha olefin feed stream comprises the aliphatic paraffin hydrocarbon solvent, such as n-heptane, and 1-hexene.

10. The method of claim 9, wherein the linear alpha olefin feed stream comprises about 3% by weight or less of C5 or smaller hydrocarbons other than ethylene.

11. A system for preparing and purifying a linear alpha olefin product, comprising:
(a) an ethylene oligomerization reactor, the reactor producing an effluent comprising the linear alpha olefin product and ethylene;
(b) a distillation column in fluid communication with the effluent of the ethylene oligomerization reactor at a feed stage, the distillation column having a plurality of stacked stages positioned between an overhead outlet and a bottoms reboiler including a stripping section of the distillation column between the feed stage and the bottoms reboiler and a rectifying section between the feed stage and the overhead outlet;
(c) an aliphatic paraffin hydrocarbon solvent source in fluid communication with the rectifying section of the distillation column; and
(d) at least one heating device positioned to add heat to at least one stage in the stripping section of the distillation column between the feed stage and the bottoms reboiler.

12. The system of claim 11, wherein the heating device comprises one or more side reboilers, each reboiler operatively positioned to receive and at least partially vaporize a liquid side stream from a different stage of the stripping section of the distillation column and return a vapor-containing effluent to the stripping section of the distillation column.

13. The system of claim 12, wherein the number of side reboilers is between 2 and 6.

14. The system of claim 11, wherein the stages of the distillation column have a first diameter in the rectifying section and a second diameter in the stripping section of the distillation column, wherein the second diameter is larger than the first diameter, such as wherein the second diameter is no more than five times the first diameter.

15. The system of claim 11, wherein an overhead stream from the distillation column comprises about 90% by weight ethylene or greater, such as about 95% by weight ethylene or greater; and/or the effluent from the ethylene oligomerization reactor comprises the aliphatic paraffin hydrocarbon solvent and 1-hexene; and/or the effluent from the ethylene oligomerization reactor comprises about 3% by weight or less of C5 or smaller hydrocarbons other than ethylene.
